# EUROPEAN PATENT APPLICATION

(11) **EP 4 516 361 A1**
(43) Date of publication of application: **05.03.2025**
(21) Application number: 23796152.9
(22) Date of filing: 14.04.2023
(51) Int. Cl.: A61Q 1/00, A61Q 19/00, A61K 8/49

(54) **COSMETIC**

(30) Priority: 25.04.2022 JP 2022071758
(71) Applicant: Shiseido Company, Ltd., Chuo-ku Tokyo 104-0061 (JP)
(72) Inventor: FUNAYAMA Mayu, Tokyo 104-0061 (JP); MURAOKA Naomi, Tokyo 104-0061 (JP); NAKAI Hitomi, Tokyo 104-0061 (JP)
(74) Representative: Ter Meer Steinmeister & Partner
(86) International application number: PCT/JP2023/015171
(87) International publication number: WO 2023/210409

(57) **Abstract**

The present invention provides a cosmetic capable of achieving both an effect brought about by nicotinic acid or a derivative thereof and a feeling of use. A powder solid cosmetic according to the present invention is a powder solid cosmetic containing a powder of nicotinic acid or a derivative thereof and an inorganic spherical powder. The powder of nicotinic acid or a derivative thereof preferably has an average particle diameter of 40 µm or less.

## Description

### TECHNICAL FIELD

The present invention relates to a cosmetic.

### BACKGROUND ART

Niacinamide is known to be an effective component for improving stains and wrinkles. In addition, niacinamide is also known to be effective as a blood circulation promoter and a hair activator. Therefore, niacinamide is widely used in various cosmetics. Niacinamide generally has a powder shape, but has high solubility in water or ethanol, and therefore is often dissolved in a liquid base to be used in a cosmetic.

Meanwhile, some cosmetics are in a formulation form that hardly contains a solvent or the like, such as a powder foundation or a concealer containing powder. It is difficult to blend niacinamide in a dissolved state in such a cosmetic. Therefore, in order to blend niacinamide in such a cosmetic, niacinamide in a solid state (powder state) is blended. However, according to studies of the present inventors, it has been found that a cosmetic containing niacinamide in a solid state, which has been generally used heretofore, has a poor feeling of use when brought into contact with the skin. The same applies to cosmetics in which powders other than niacinamide are blended, and it has been found that even when niacinamide in a solid state is blended in a cosmetic in which a silica powder or a resin powder is blended, a feeling of use tends to deteriorate.

Even in prior art, a cosmetic containing niacinamide in a solid state is hardly known. As far as the present inventors know, there are only several known cosmetics in which niacinamide is blended in a very small amount of about 0.1% by weight with respect to the total mass of a cosmetic (for example, Patent Literature 1).

### CITATION LIST

### PATENT LITERATURE

Patent Literature 1: JP 2010-503724 A

### SUMMARY OF THE INVENTION

The present invention provides the following inventions.
[1] A powder solid cosmetic containing:
   a powder of nicotinic acid or a derivative thereof; and
   inorganic spherical particles.
[2] The cosmetic according to claim 1, in which the powder of nicotinic acid or a derivative thereof has an average particle diameter of 40 µm or less.
[3] The cosmetic according to [1] or [2], in which the content of the powder of nicotinic acid or a derivative thereof is 3% by mass or more based on the total mass of the cosmetic.
[4] The cosmetic according to any one of [1] to [3], in which the nicotinic acid or a derivative thereof is selected from nicotinic acid, niacinamide, benzyl nicotinate, tocopherol nicotinate, and a nicotinic acid β-butoxy ester.
[5] The cosmetic according to any one of [1] to [4], in which the inorganic spherical powder is inorganic spherical silica.
[6] The cosmetic according to any one of [1] to [5], in which the inorganic spherical powder has an average particle diameter of 1 to 20 µm.
[7] The cosmetic according to any one of [1] to [6], in which the inorganic spherical powder has a specific surface area of 100 to 500 m²/g.
[8] The cosmetic according to any one of [1] to [7], in which the content of water is 1% by mass or less based on the total mass of the cosmetic.

By containing a powder of nicotinic acid or a derivative thereof and an inorganic spherical powder, the solid cosmetic according to the present invention can improve usability such as excellent smoothness while having an effect brought about by nicotinic acid or a derivative thereof in a cosmetic containing almost no liquid, such as powder, and further improves "payoff" by an application tool such as a puff.

### DETAILED DESCRIPTION OF THE INVENTION

### [Powder solid cosmetic]

A powder solid cosmetic (hereinafter, also simply referred to as a "cosmetic") according to the present invention contains a powder of nicotinic acid or a derivative thereof and an inorganic spherical powder. Hereinafter, components of the cosmetic according to the present invention will be described.

Examples of the derivative of nicotinic acid include niacinamide, benzyl nicotinate, tocopherol nicotinate, and a nicotinic acid β-butoxy ester, and niacinamide is preferable among these derivatives. Here, niacinamide is also referred to as nicotinamide or niacinamide, and is an amide compound of nicotinic acid (vitamin B3/niacin). Niacinamide is a known substance that is a water-soluble vitamin belonging to a vitamin B group, may be extracted from a natural product (rice bran or the like) or can be synthesized by a known method. For example, those listed in the Japanese Pharmacopoeia, Eighteenth Edition can be used.

In general, nicotinic acid or a derivative thereof is blended in a cosmetic in a state of being dissolved in a solvent such as water. On the other hand, in the cosmetic according to the present invention, nicotinic acid or a derivative thereof is blended in a powder state. Here, nicotinic acid or a derivative thereof, for example, niacinamide used industrially is generally commercially available in a particle form. This is considered to be because when niacinamide has an excessively small average particle diameter, scattering or the like is likely to occur, and handleability is deteriorated. Specifically, niacinamide used industrially generally has an average particle diameter of more than 40 µm.

On the other hand, the average particle diameter of nicotinic acid or a derivative thereof used in the present invention is not particularly limited, but is preferably 40 µm or less, more preferably 30 µm or less, and particularly preferably 15 µm or less. By using nicotinic acid or a derivative thereof having such a small average particle diameter, the cosmetic according to the present invention can achieve both an effect of nicotinic acid or a derivative thereof as a drug and an excellent feeling of use. A material having such a small average particle diameter can be obtained by pulverizing a commercially available material (details will be described later). Note that, in the present invention, a material before pulverization may be referred to as a particle, and a material having a small average particle diameter after pulverization may be referred to as a powder.

In the present invention, the average particle diameter of a powder can be measured by a laser diffraction/scattering method. The "average particle diameter" in the present invention is a particle diameter at which a cumulative frequency is 50% from a number-based particle size distribution curve measured by a laser diffraction/scattering method, particularly a microtrack laser diffraction method. In the microtrack laser diffraction method, scattered light when powder particles are irradiated with laser light is used.

More specifically, the average particle diameter is measured by a wet method in which a solvent that does not dissolve nicotinic acid or a derivative thereof, for example, niacinamide, for example, a solvent such as an isoparaffinic hydrocarbon solvent (for example, ISOPAR (trade name, manufactured by Exxon Mobil Corporation) or D-5 is circulated, and a powder sample is put into the solvent and irradiated with laser light. The measurement can be performed using, for example, Microtrac MT-3000 manufactured by Nikkiso Co., Ltd. According to this method, in general, measurement can be performed for a powder having an average particle diameter of 0.1 µm to 200 µm.

A blending ratio of nicotinic acid or a derivative thereof contained in the cosmetic of the present invention is not particularly limited, but is preferably 3% by mass or more, and particularly preferably 5% by mass or more with respect to the total mass of the cosmetic. In general, when nicotinic acid or a derivative is used for a cosmetic in a form of particles or powder, a feeling of use tends to be impaired. However, by using nicotinic acid or a derivative thereof having the average particle diameter specified in the present invention, a sufficiently satisfactory feeling of use can be achieved even when the blending ratio is high. Meanwhile, although an upper limit is not particularly limited, it is difficult to obtain an effect proportional to the addition amount even when the blending ratio is excessively increased. Therefore, the blending ratio of nicotinic acid or a derivative thereof is preferably 10% by mass or less, and particularly preferably 8% by mass or less with respect to the total mass of the cosmetic.

The cosmetic according to the present invention generally contains an inorganic powder different from nicotinic acid or a derivative thereof, and further contains an inorganic spherical powder as at least a part thereof. In the present invention, the inorganic spherical powder is a powder having an aspect ratio of 2 or less, preferably 1.5 or less, and is most preferably a true spherical powder among inorganic powders containing an inorganic material as a main component.

Examples of the inorganic powder include silica (anhydrous silicic acid), talc, boron nitride, sericite, natural mica, calcined mica, synthetic mica, synthetic sericite, alumina, mica, kaolin, bentonite, smectite, calcium carbonate, magnesium carbonate, calcium phosphate, magnesium oxide, tin oxide, iron oxide, yttrium oxide, chromium oxide, titanium oxide, zinc oxide, cerium oxide, aluminum oxide, magnesium oxide, chromium hydroxide, Prussian blue, ultramarine blue, calcium phosphate, aluminum hydroxide, barium sulfate, magnesium sulfate, silicic acid, aluminum magnesium silicate, calcium silicate, barium silicate, magnesium silicate, aluminum silicate, strontium silicate, calcium aluminum borosilicate, silicon carbide, magnesium fluoride, a metal tungstate, magnesium aluminate, magnesium aluminometasilicate, chlorhydroxyaluminum, clay, zeolite, hydroxyapatite, ceramic powder, spinel, mullite, cordierite, aluminum nitride, titanium nitride, silicon nitride, lanthanum, samarium, tantalum, terbium, europium, neodymium, Mn-Zn ferrite, Ni-Zn ferrite, silicone carbide, cobalt titanate, barium titanate, iron titanate, lithium cobalt titanate, cobalt aluminate, antimony-containing tin oxide, tin-containing indium oxide, magnetite, aluminum powder, gold powder, silver powder, platinum powder, copper powder, noble metal colloid, iron powder, zinc powder, cobalt blue, cobalt violet, cobalt green, low order titanium oxide, fine particle titanium oxide, butterfly-shaped barium sulfate, petal-shaped zinc oxide, tetrapod-shaped zinc oxide, fine particle zinc oxide, and as pearl pigments, titanium oxide-coated mica, titanium oxide-coated mica, titanium oxide-coated synthetic mica, titanium oxide-coated silica, titanium oxide-coated synthetic mica, titanium oxide-coated talc, zinc oxide-coated silica, titanium oxide-coated colored mica, red iron oxide-coated titanated mica, red iron oxide/black iron oxide-coated titanated mica, carmine-coated titanated mica, and blue iron-coated titanated mica.

Furthermore, as an inorganic powder functioning as a coloring material, an inorganic red pigment (for example, iron oxide or iron titanate), an inorganic brown pigment (for example, γ-iron oxide), an inorganic yellow pigment (for example, yellow iron oxide or yellow earth), an inorganic black pigment (for example, black iron oxide or low order titanium oxide), an inorganic violet pigment (for example, mango violet or cobalt violet), an inorganic green pigment (for example, chromium oxide, chromium hydroxide, or cobalt titanate), an inorganic blue pigment (for example, ultramarine blue or Prussian blue), a pearl pigment (for example, bismuth oxychloride, fish scale foil, or titanated mica), a metal powder pigment (for example, aluminum powder or copper powder), and the like can also be used. An organic powder can also be used as a coloring material according to a purpose. Examples of such an organic powder include organic pigments of zirconium, barium, and aluminum lake (for example, Red No. 201, Red No. 202, Red No. 204, Red No. 205, Red No. 220, Red No. 226, Red No. 228, Red No. 405, Orange No. 203, Orange No. 204, Yellow No. 205, Yellow No. 401, Blue No. 404, Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, and Blue No. 1).

Among these components, silica, talc, mica, sericite, kaolin, titanium oxide, iron oxide, zinc oxide, and the like are preferable, and silica is particularly preferable. This is because when silica and pulverized nicotinic acid or a derivative thereof are combined in a cosmetic containing no solvent, an effect of improving usability tends to be large, and an effect of improving "payoff" is large. This improving effect is strongly exhibited also in a cosmetic in which a resin powder is used as a raw material or removed. These inorganic powders may be subjected to a surface treatment, and are particularly preferably subjected to a hydrophobic treatment. In such a hydrophobic treatment, for example, a hydrophobic treatment agent such as a fatty acid, a higher fatty acid, a higher alcohol, a hydrocarbon, a triglyceride, an ester, dimethicone, a silicone resin, or a fluorine compound is used.

In addition, these inorganic powders can have various particle shapes, but the cosmetic according to the present invention contains a spherical inorganic powder. In particular, as the silica, many spherical silicas having a spherical shape are known, and it is preferable to use these spherical silicas.

The average particle diameter of the inorganic spherical powder can be appropriately adjusted from a viewpoint of usability or the like. Specifically, the average particle diameter is preferably 1 to 20 µm, and more preferably 2 to 10 µm. Here, the average particle diameter is measured by a method similar to the above-described method for measuring the average particle diameter of nicotinic acid or a derivative thereof.

In addition, inorganic particles have various surface states, and porous or non-porous inorganic particles are known. One parameter expressing such a surface shape is a specific surface area per unit mass. The specific surface area of the inorganic spherical powder used in the present invention is not particularly limited, but as the specific surface area is higher, the effect of improving "payoff" tends to be larger. Specifically, the specific surface area is preferably 100 to 500 m²/g, and more preferably 150 to 300 m²/gg. Here, the specific surface area per unit mass is measured by a nitrogen adsorption method known as a Brunauer-Emmett-Teller method (BET method) described in The Journal of the American Chemical Society, vol. 60, page 309 (February 1938), which corresponds to International Standard ISO5794/1 (Appendix D).

The cosmetic according to the present invention contains an inorganic spherical powder, but in addition, particles having a plate shape or a needle shape can also be used within a range in which the effect of the present invention is not impaired, and it is preferable to combine these particles while considering usability and the like.

A blending ratio of the inorganic powder in the cosmetic according to the present invention is preferably 20 to 80% by mass, and more preferably 30 to 70% by mass based on the total mass of the cosmetic. In addition, a blending ratio of the inorganic spherical powder as a part of the inorganic powder is preferably 2 to 20% by mass, and more preferably 2 to 10% by mass based on the total mass of the cosmetic.

In addition, as described above, the cosmetic according to the present invention can achieve excellent usability even when not containing a resin powder generally used for improving usability, but can contain a resin powder or an organic powder as necessary. For example, the organic pigment described above can be preferably used because it can achieve coloring suitable for a cosmetic, and a cellulose powder, starch, and an amino acid powder (for example, lauroyl lysine) can be preferably used because they can further improve usability.

The cosmetic according to the present invention is a powder solid cosmetic containing nicotinic acid or a derivative thereof, an inorganic spherical powder, and if necessary, an inorganic powder other than the spherical powder as main components. The powder solid cosmetic is a cosmetic which is made easy to handle by suppressing scattering of a powder, and is obtained by solidifying the powder with a binder or the like. As the binder, an oil is generally used.

The oil that can be used in the cosmetic according to the present invention is not particularly limited as long as it can be generally used in manufacture of a cosmetic. Specific examples thereof include a liquid oil and fat (for example, macadamia nut oil, avocado oil, or camellia oil), a solid oil and fat (for example, cocoa butter, coconut oil, horse fat, or hardened coconut oil), a wax (for example, beeswax, candelilla wax, cotton wax, carnauba wax, or bayberry wax), a hydrocarbon oil (for example, liquid paraffin, ozokerite, or squalane), a higher fatty acid (for example, lauric acid, myristic acid, palmitic acid, stearic acid, or behenic acid), a higher alcohol (lauryl alcohol, cetyl alcohol, stearyl alcohol, behenyl alcohol, or butyl alcohol), a synthetic ester oil (for example, isopropyl myristate, cetyl octanoate, octyldodecyl myristate, isopropyl palmitate, or neopentyl glycol dicaprate), and a silicone oil (for example, dimethylpolysiloxane, methylhydrogenpolysiloxane, methylphenylpolysiloxane, or stearoxymethylpolysiloxane).

When an oil is blended, a blending ratio thereof is suitably 1 to 20% by mass, and preferably 5 to 10% by mass with respect to the total mass of the cosmetic.

In addition, the cosmetic according to the present invention can contain other components as long as the effect of the present invention is not impaired. Examples of the other components include an antiseptic (for example, ethylparaben, butylparaben, chlorphenesin, or phenoxyethanol), a dispersant (various surfactants), an antioxidant (for example, tocopherols, dibutylhydroxytoluene, butylhydroxyanisole, or gallic acid esters), a fragrance, a moisturizer, a water-soluble polymer, a thickener, a coating agent, an ultraviolet absorber, a sequestering agent, a lower alcohol, a polyhydric alcohol, a sugar, an amino acid, an organic amine, a polymer emulsion, a pH adjuster, a skin nutrient, a vitamin, and water.

Note that, in the cosmetic according to the present invention, the content of water is particularly preferably zero in order to maximize the effect obtained by nicotinic acid or a derivative thereof blended as a powder. Therefore, when water is blended as necessary, a blending ratio thereof is preferably 1% by mass or less, and more preferably 0.5% by mass or less based on the total mass of the cosmetic.

Specifically, the powder solid cosmetic according to the present invention is more preferably in a form of a foundation, an eye shadow, a cheek color, a pressed powder, a deodorant powder, a face powder, or the like.

### [Method for manufacturing powder solid cosmetic]

The powder solid cosmetic according to the present invention can be manufactured by any method. In general, the powder solid cosmetic is manufactured by mixing components, but a cosmetic containing particles of nicotinic acid or a derivative thereof having a specific particle diameter can be manufactured by pulverizing particles of nicotinic acid or a derivative thereof having a large average particle diameter to form a powder having a specific particle diameter, for example, an average particle diameter of 40 µm or less.

When the particles are pulverized, only nicotinic acid or a derivative thereof may be pulverized, or a mixture containing other components to be blended in the cosmetic, for example, inorganic particles may be pulverized. For the pulverization, a stirring mixer or a pulverizing machine, such as a Henschel mixer, an atomizer, a Nauta mixer, a ribbon blender, a kneader, or a pulverizer can be used.

When only particles of nicotinic acid or a derivative thereof, for example, only niacinamide particles are pulverized to form a powder, it is preferable to use an atomizer or the like capable of forming a powder having a relatively small average particle diameter. In addition, it is also preferable to use an atomizer after pulverization with a Henschel mixer or the like capable of forming a powder having a relatively large average particle diameter.

When pulverization is performed after nicotinic acid or a derivative thereof is mixed with components, it is common to first use a Henschel mixer for pulverization. Since an inorganic powder and the like are blended as other components, nicotinic acid or a derivative thereof tends to have an average particle diameter smaller by contact with the inorganic powder than that of a case in which nicotinic acid or a derivative thereof is pulverized alone. However, when the average particle diameter of nicotinic acid or a derivative thereof does not become a specific particle diameter, for example, 40 µm or less, it is preferable to further pulverize nicotinic acid or a derivative thereof with an atomizer or the like. Note that the average particle diameter of all the materials contained in the pulverized cosmetic does not need to be, for example, 40 µm or less. Even when the average particle diameter of components other than nicotinic acid or a derivative thereof exceeds 40 µm, an influence on a feeling of use is small.

When an oil is blended as a component to form a powder solidified cosmetic, it is also possible to blend the materials stepwise in addition to mixing and pulverizing all the materials first. Particularly preferably, the pulverization step is divided into two or more steps, and an oil is blended after one or more pulverization steps are performed. Specifically, a mixture obtained by mixing and pulverizing nicotinic acid or a derivative thereof, an inorganic powder, and if necessary, a component other than an oil among components of other materials with a Henschel mixer, and then mixing the oil can be pulverized with an atomizer. Through such a step, a uniform powder cosmetic containing no coarse particles can be obtained. The powder cosmetic after the pulverization can be compressed by a compressor or the like to form a powder solid cosmetic.

### EXAMPLES

### [Example 1 to 4 and Comparative Example 1 to 3]

A powder solid cosmetic (pressed powder cosmetic) was prepared with a formulation presented in Table 1, and performance thereof was evaluated. Note that a numerical value in Table is a blending ratio based on the total mass of a cosmetic. Evaluation criteria are as follows.

The powder solid cosmetics of the examples were actually used and evaluated for "payoff" and "smoothness" by ten expert panels. Specifically, the amount of the cosmetic attached to a puff when the puff was used and smoothness when the cosmetic was applied to the skin were evaluated in three grades.
A: Seven or more out of ten expert panels evaluated that it was "excellent".
B: Five or more out of ten expert panels evaluated that it was "excellent".
C: Four or less out of ten expert panels evaluated that it was "excellent".

The obtained results are as presented in Table 1.

### [Table 1]

**Table 1**

| | Comparative Example | | | Example | | | |
|---|---|---|---|---|---|---|---|
| | 1 | 2 | | 1 | 2 | 3 | 4 |
| Niacinamide (average particle diameter: 40 µm or less) | | 5.00% | 5.00% | 5.00% | 5.00% | 5.00% | 5.00% |
| Spherical silica | | | | 2.00% | | | |
| (average particle diameter: 5 µm, specific surface area: 300 m²/g) | | | | | | | |
| Spherical silica | | | | | | 3.00% | |
| (average particle diameter: 2 to 4 µm, specific surface area: 300 m²/g) | | | | | | | |
| Spherical silica | | | | | | | 3.00% |
| (average particle diameter: 6 µm, specific surface area: 170 m²/g) | | | | | | | |
| Spherical silica (average particle diameter: 9 µm, specific surface area: 160 m²/g) | | | | | 3.00% | | |
| Talc | 19.15% | 9.15% | 6.15% | 7.15% | 6.15% | 6.15% | 6.15% |
| Hydrophobic treatment talc | 10.00% | 10.00% | 10.00% | 10.00% | 10.00% | 10.00% | 10.00% |
| Sericite | | 5.00% | 5.00% | 5.00% | 5.00% | 5.00% | 5.00% |
| Synthetic phlogopite | 20.00% | 20.00% | 20.00% | 20.00% | 20.00% | 20.00% | 20.00% |
| Methyl methacrylate crosspolymer | 15.00% | 15.00% | 18.00% | 15.00% | 15.00% | 15.00% | 15.00% |
| (Vinyldimethicone/Methiconesilsesquioxane) crosspolymer | 5.00% | 5.00% | 5.00% | 5.00% | 5.00% | 5.00% | 5.00% |
| Titanium oxide | 8.00% | 8.00% | 8.00% | 8.00% | 8.00% | 8.00% | 8.00% |
| Pigment-grade titanium oxide | 8.00% | 8.00% | 8.00% | 8.00% | 8.00% | 8.00% | 8.00% |
| Red iron oxide | 0.92% | 0.92% | 0.92% | 0.92% | 0.92% | 0.92% | 0.92% |
| Yellow iron oxide | 1.69% | 1.69% | 1.69% | 1.69% | 1.69% | 1.69% | 1.69% |
| Black iron oxide | 0.11% | 0.11% | 0.11% | 0.11% | 0.11% | 0.11% | 0.11% |
| Chlorphenesin | 0.20% | 0.20% | 0.20% | 0.20% | 0.20% | 0.20% | 0.20% |
| Tocopherol | 0.02% | 0.02% | 0.02% | 0.02% | 0.02% | 0.02% | 0.02% |
| Ethylhexyl methoxycinnamate | 5.00% | 5.00% | 5.00% | 5.00% | 5.00% | 5.00% | 5.00% |
| Diphenylsiloxy phenyl trimethicone | 2.00% | 2.00% | 2.00% | 2.00% | 2.00% | 2.00% | 2.00% |
| Neopentyl glycol dicaprate | 2.41% | 2.41% | 2.41% | 2.41% | 2.41% | 2.41% | 2.41% |
| Triethylhexanoin | 1.50% | 1.50% | 1.50% | 1.50% | 1.50% | 1.50% | 1.50% |
| Sorbitan sesquiisostearate | 1.00% | 1.00% | 1.00% | 1.00% | 1.00% | 1.00% | 1.00% |
| Total | 100.00% | 100.00% | 100.00% | 100.00% | 100.00% | 100.00% | 100.00% |
| Payoff | A | C | C | A | B | A | B |
| Smoothness | A | B | B | A | B | A | B |

### [Formulation Example]

Hereinafter, a formulation example suitable as the cosmetic of the present invention will be presented. The blending amounts are all expressed by *%* by mass.

### [Table 2]

**Table 2**

| | |
|---|---|
| Niacinamide | 5.00% |
| (average particle diameter: 40 µm or less) | |
| Spherical silica | 3.00% |
| (average particle diameter: 2 to 4 µm, specific surface area: 300 m²/g) | |
| Talc | 26.15% |
| Hydrophobic treatment talc | 10.00% |
| Sericite | 5.00% |
| Synthetic phlogopite | 20.00% |
| Titanium oxide | 8.00% |
| Pigment-grade titanium oxide | 8.00% |
| Red iron oxide | 0.92% |
| Yellow iron oxide | 1.69% |
| Black iron oxide | 0.11% |
| Chlorphenesin | 0.20% |
| Tocopherol | 0.02% |
| Ethylhexyl methoxycinnamate | 5.00% |
| Diphenylsiloxy phenyl trimethicone | 2.00% |
| Neopentyl glycol dicaprate | 2.41% |
| Triethylhexanoin | 1.50% |
| Sorbitan sesquiisostearate | 1.00% |
| Total | 100.00% |

## Claims

1. A powder solid cosmetic comprising:
a powder of nicotinic acid or a derivative thereof; and
an inorganic spherical powder.

2. The cosmetic according to claim 1, wherein the powder of nicotinic acid or a derivative thereof has an average particle diameter of 40 µm or less.

3. The cosmetic according to claim 1 or 2, wherein a content of the powder of nicotinic acid or a derivative thereof is 3% by mass or more based on a total mass of the cosmetic.

4. The cosmetic according to claim 1 or 2, wherein the nicotinic acid or a derivative thereof is selected from nicotinic acid, niacinamide, benzyl nicotinate, tocopherol nicotinate, and a nicotinic acid β-butoxy ester.

5. The cosmetic according to claim 1 or 2, wherein the inorganic spherical powder is inorganic spherical silica.

6. The cosmetic according to claim 1 or 2, wherein the inorganic spherical powder has an average particle diameter of 1 to 20 µm.

7. The cosmetic according to claim 1 or 2, wherein the inorganic spherical powder has a specific surface area of 100 to 500 m²/g.

8. The cosmetic according to claim 1 or 2, wherein a content of water is 1% by mass or less based on a total mass of the cosmetic.
